(19) Europäisches Patentamt
European Patent Office
Office européen des brevets

(11) Veröffentlichungsnummer: 0 358 152
A1

(12) **EUROPÄISCHE PATENTANMELDUNG**

(21) Anmeldenummer: 89116317.2

(22) Anmeldetag: 04.09.89

(51) Int. Cl.5: C07D 239/26 , C07D 239/42 , C07D 241/12 , C07D 237/08 , C07D 239/46 , A61K 31/505 , A61K 31/495 , A61K 31/50 , C25B 3/10

(30) Priorität: 02.09.88 DD 319459

(43) Veröffentlichungstag der Anmeldung: 14.03.90 Patentblatt 90/11

(84) Benannte Vertragsstaaten: CH DE FR GB IT LI NL

(71) Anmelder: Humboldt-Universität zu Berlin
Unter den Linden 6
DDR-1086 Berlin(DD)

(72) Erfinder: Hess, Ulrich, Doz. Dr.sc.nat., Dipl.-Chem.
Hartriegelstrasse 19
DDR-Berlin 1190(DD)
Erfinder: Huhn, Dietmar, Dr.rer.nat., Dipl.-Chem.
Mittelstrasse 15 a
DDR-Berlin 1125(DD)
Erfinder: Neubert-Hils,Jutta,Dr.sc.med.,Dr.rer.nat.
Karl-Marx-Allee 26
DDR-Berlin 1020(DD)
Erfinder: May, Annelies, Dr.rer.nat.
Schlichtingheimer Weg 5
DDR-Berlin 1183(DD)

(74) Vertreter: Spott, Gottfried, Dr. et al
Patentanwälte Spott und Puschmann
Sendlinger-Tor-Platz 11
D-8000 München 2(DE)

(54) **Diazinsubstituierte 1-Adamantane, Verfahren zu ihrer Herstellung und ihre Verwendung als Arzneimittel.**

(57) Beschrieben werden neue diazinsubstituierte 1-Adamantane der Formel

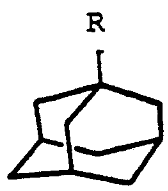

(I)

,

worin R für einen unsubstituierten oder einen beispielsweise durch Alkyl, Alkoxy, Aryl, Amino, Acylamino, Hydroxy, Adamant-1-yl, Halogen oder Pseudohalogen substituierten Pyridazinring, Pyrazinring oder Pyrimidinring steht. Diese Verbindungen können beispielsweise durch elektrochemische Synthese in einem Reaktionsschritt aus 1-Halogenadamantanen und entsprechenden Pyridazinen, Pyrazinen oder Pyrimidinen in einem aprotischen Lösungsmittel und in Anwesenheit eines Leitsalzes hergestellt werden. Sie finden beispielsweise als Arzneimittel Anwendung und zeichnen sich durch eine interessante virostatische Wirksamkeit und vor allem eine Wirksamkeit gegen Influenzaviren aus.

EP 0 358 152 A1

Xerox Copy Centre

# Diazinsubstituierte 1-Adamantane, Verfahren zu ihrer Herstellung und ihre Verwendung als Arzneimittel

Die Erfindung betrifft neue diazinsubstituierte 1-Adamantane, ein Verfahren zu ihrer Herstellung und ihre Verwendung als Arzneimittel. Sie ist in der pharmazeutischen Industrie sowie in der Humanmedizin und der Veterinärmedizin einsetzbar.

Es ist bekannt, daß in Stellung 1 substituierte Adamantane verschiedene pharmakologische Wirkungen aufweisen. Heterocyclisch funktionalisierte 1-Adamantane, wie 2-(Adamant-1-yl)-uracil oder 2-(Adamant-1-yl)-cytosin (J. Med. Chem. 18, Seite 713 (1975)) sowie 4(6)-(Adamant-1-yl)-pyrimidin (J. Chem. Educ. 50, Seite 780 (1973)) und 5-(Adamant-1-yl)-pyrimidin (JP-A 71 04 370) zeigen virostatische Aktivität gegenüber Newcastle Viren, wobei für die letztgenannte Verbindung auch eine immunsuppressive und cancerostatische Wirkung angegeben wird. Eine Aktivität dieser Verbindungen gegenüber Influenzaviren ist im Gegensatz zu einer solchen Wirkung von 1-Aminoadamantan und 1-Aminomethyladamantan und ihren N-substituierten Derivaten (J. Med. Chem. 14, Seite 535 (1971), J. Med. Chem. 6, Seite 760 (1963) und Latv. RGR Akad. Vestis, Seite 32 (1972)) nicht bekannt.

Diazinsubstituierte 1-Adamantane der oben genannten Strukturen werden vorwiegend durch mehrstufige Ringschlußreaktionen aus einer den 1-Adamantylrest enthaltenden Komponente und dem entsprechenden Cyclisierungssynthon hergestellt (J. Med. Chem. 13, Seite 1170 (1970), J. Med. Chem. 14, Seite 408 (1971), J. Med. Chem. 15, Seite 6621 (1972), DE-A 2 142 385), wobei gegebenenfalls die Reaktion trimethylsilyloxylierter Zwischenstufen durch Substitution mittels 1-Chloradamantan in Gegenwart von Lewis-Säuren zum Zielprodukt führt (J. Org. Chem. 45, Seite 3559 (1980) und Chem. Pharm. Bull. 30, Seite 2051 (1982)).

4-(Adamant-1-yl)-pyrimidin oder 4-(Adamant-1-yl)-5-methylpyrimidin, von denen eine pharmakologische Wirkung nicht beschrieben ist, werden durch Umsetzung von 3-(Adamant-1-yl)-3-chloracrylaldehyd oder von 3-(Adamant-1-yl)-3-chlor-methacrylaldehyd und Formamidin hergestellt (Isv. Akad. Nayk SSSR Ser. Chim. 8, Seiten 1858 und 2549 (1985)). In Stellung 2 adamantyliertes Uracil oder Cytosin wird durch Umsetzung von Adamant-1-yl-formamidin mit Dimethylmalonat oder Cyanessigsäure unter Ringschlußreaktion hergestellt (J. Med. Chem. 18, Seite 713 (1975)). Pyrazinsubstituierte 1-Adamantane sind nur in Form benzokondensierter Vertreter (Chinoxaline) bekannt. 2-(Adamant-1-yl)-chinoxalin wird aus Adamant-1-ylglyoxal und o-Diaminobenzen (BUll. Chem. Soc. Japan 116, Seite 1617 (1969)) durch chemische Ringschlußsynthese hergestellt, während die Herstellung von 2-(Adamant-1-yl)-3-ethylchinoxalin und von 2-(Adamant-1-yl)-3-benzylchinoxalin aus entsprechenden 1,2-dicarbonylsubstituierten 1-Adamantanen (Rec. Trav. Chim. Pays-Bas 104, Seite 50 (1985)) erfolgt. Über pharmakologische Wirkungen dieser chinoxalinsubstituierten 1-Adamantane wird jedoch nirgends berichtet. Pyridazinsubstituierte 1-Adamantane sind bisher überhaupt nicht bekannt. Alle Verfahren zur Herstellung dieser Verbindungen sind im übrigen ziemlich aufwendig, weil nach ihnen die Adamant-1-ylfunktion über mehrstufig herzustellende Ausgangsstoffe eingeführt werden muß. Direkte Substitutionen mit Halogenadamantan an Diazinen gibt es bisher nicht. Sie gelingen nur an den perhydrierten Analoga dieser Heterocyclen und führen ausnahmslos zu N-(Adamant-1-yl)-derivaten (C. A. 88, Referat 136675y (1978)).

Aufgabe der Erfindung ist nun die Schaffung neuer diazinsubstituierter 1-Adamantane, eines Verfahrens zur Herstellung solcher Verbindungen und ihre Verwendung als Arzneimittel, vor allem als virostatische Mittel, und insbesondere als Mittel zur Hemmung von Influenzaviren.

Diese Aufgabe wird erfindungsgemäß gelöst durch diazinsubstituierte 1-Adamantane der allgemeinen Formel I

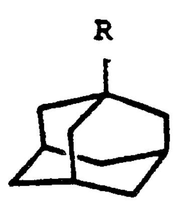

(I) ,

worin R entweder für einen unsubstituierten oder einen einfach in möglichen Positionen durch Alkyl, Alkoxy, Aryl, Amino, Acylamino, Hydroxy, Adamant-1-yl, Halogen oder Pseudohalogen substituierten oder für einen mehrfach in möglichen Positionen gleich oder verschieden durch Alkyl, Alkoxy, Aryl, Amino, Acylamino, Hydroxy, Halogen oder Pseudohalogen substituierten oder einen mehrfach in möglichen Positionen gleich

oder verschieden durch Alkyl, Alkoxy, Aryl, Amino, Acylamino, Hydroxy, Halogen oder Pseudohalogen und durch Adamant-1-yl substituierten Pyridazinrest oder Pyrazinrest steht,

oder worin R einen unsubstituierten oder einen einfach in möglichen Positionen durch Alkoxy, Aryl, Acylamino, Halogen oder Pseudohalogen oder durch 2-Amino, 2- oder 4(6)-Alkyl oder 2- oder 4(6)-(Adamant-1-yl) substituierten oder für einen mehrfach in möglichen Positionen gleich oder verschieden durch Alkyl, Alkoxy, Aryl, Acylamino, Halogen oder Pseudohalogen substituierten oder einen mehrfach in möglichen Positionen gleich oder verschieden durch Alkyl, Alkoxy, Aryl, Acylamino, Halogen oder Pseudohalogen und durch Adamant-1-yl substituierten Pyrimidinrest bedeutet.

Diese diazinsubstituierten 1-Adamantane weisen interessante pharmakologische Eigenschaften auf, und sie verfügen vor allem über eine virostatische Wirksamkeit, insbesondere eine Hemmwirkung gegenüber Influenzaviren vom Typ A und/oder vom Typ B.

Bei den erfindungsgemäßen diazinsubstituierten 1-Adamantanen der allgemeinen Formel (I) ist bevorzugt, daß die möglichen Positionen der Substitution im Pyridazinrest die Kohlenstoffatome 3 und/oder 4 und/oder 5 und/oder 6, im Pyrazinrest die Kohlenstoffatome 2 und/oder 3 und/oder 5 und/oder 6 und im Pyrimidinrest die Kohlenstoffatome 2 und/oder 4 und/oder 5 und/oder 6 sind, daß Alkyl jeweils für unverzweigtes $C_nH_{2n+1}$ mit n gleich 1 bis 4 steht, daß Alkoxy jeweils unverzweigtes $C_nH_{2n+1}O$- mit n gleich 1 bis 4 bedeutet, daß Aryl jeweils Phenyl oder durch einen oder mehrere Elektronendonatoren substituiertes Phenyl ist, daß Amino für $H_2N$-, $CH_3NH$-, $C_2H_5NH$-, $(CH_3)_2N$- oder $(C_2H_5)_2N$-, daß Acylamino für $CH_3CO$ NH-, $C_2H_5CONH$-, $C_6H_5CONH$- oder $C_6H_5CH_2CONH$- steht, daß Halogen Fluor, Chlor, Brom oder Iod ist, und daß Pseudohalogen Cyano bedeutet.

Erfindungsgemäß bevorzugte diazinsubstituierte 1-Adamantane sind Verbindungen der allgemeinen Formel (I), bei denen der Substituent R für Pyridazin-4(5)-yl, Pyrazin-2(3,5,6)-yl, 2-Methylpyrazin-6(5,3)-yl, 6-(Adamant-1-yl)-pyrazin-2-yl, 5-(Adamant-1-yl)-pyrazin-2-yl, 2(3)-(Adamant-1-yl)-pyrazin-3(2)-yl, 4(6)-Methylpyrimidin-6(4)-yl, 4(6)-(Adamant-1-yl)-pyrimidin-6(4)-yl oder 2-Aminopyrimidin-6(4)-yl steht.

Die erfindungsgemäßen diazinsubstituierten 1-Adamantane der allgemeinen Formel I lassen sich beispielsweise dadurch herstellen, daß ein 1-Halogenadamantan, vorzugsweise 1-Bromadamantan, mit einem unsubstituierten oder einfach in möglichen Positionen durch Alkyl- Alkoxy, Aryl, Amino, Acylamino, Hydroxy, Adamant-1-yl, Halogen oder Pseudohalogen substituierten oder einem mehrfach in möglichen Positionen gleich oder verschieden durch Alkyl, Alkoxy, Aryl, Amino, Acylamino, Hydroxy, Halogen oder Pseudohalogen substituierten oder einem mehrfach in möglichen Positionen gleich oder verschieden durch Alkyl, Alkoxy, Aryl, Amino, Acylamino, Hydroxy, Halogen oder Pseudohalogen und durch Adamant-1-yl substituiertes Pyridazin mit freier oder mit durch Chlor, Brom oder Iod substituierter Stellung 3(6) und/oder Stellung 4(5) oder Pyrazin mit freier oder mit durch Chlor, Brom oder Iod substituierter Stellung 2 oder Pyrimidin mit freier oder mit durch Chlor, Brom oder Iod substituierter Stellung 2 und/oder Stellung 4(6) vermischt und in einem wasserfreien Lösungsmittel in Gegenwart eines Leitsalzes unter Schutzgasatmosphäre elektrochemisch umgesetzt wird.

Als Schutzgasatmosphäre wird hierbei vorzugsweise eine Stickstoffatmosphäre angewandt, und die erfindungsgemäße elektrochemische Umsetzung wird in einer üblichen Elektrolysezelle mit einem Diaphragma an einer Metallkathode durchgeführt, die beispielsweise eine Quecksilberkathode oder eine Bleikathode ist. Das Diaphragma besteht zweckmäßigerweise aus Frittenglas, einem porösen keramischen Material, einem Kunststoff oder einer Ionenaustauschermembran.

Als Reaktant zur Einführung des Restes R wird vorzugsweise Pyridazin, 3,6-Dichlorpyridazin, Pyrazin, 2-Methylpyrazin, 2-(Adamant-1-yl)-pyrazin, Pyrimidin, 4(6)-Methylpyrimidin, 2-Aminopyrimidin, 4(6)-(Adamant-1-yl)-pyrimidin oder 6-Amino-4-hydroxypyrimidin verwendet.

Das erfindungsgemäße Verfahren wird in einem wasserfreien Lösungsmittel, zweckmäßigerweise einem aprotischen organischen Lösungsmittel, durchgeführt, und als solche Lösungsmittel werden vorzugsweise Dimethylformamid, Acetonitril, Propylencarbonat oder Methanol verwendet.

Als Leitsalz wird beim erfindungsgemäßen Verfahren zweckmäßigerweise ein Tetraalkylammoniumsalz angewandt, und Beispiele für solche Leitsalze sind Tetraethylammoniumiodid, Tetraethylammoniumbromid, Tetraethylammoniumperchlorat, Tetraethylammoniumtetrafluorborat, Tetrabutylammoniumiodid, Tetrabutylammoniumbromid, Tetrabutylammoniumperchlorat oder Tetrabutylammoniumtetrafluorborat.

Das beim erfindungsgemäßen Verfahren als Ausgangsmaterial benötigte 1-Halogenadamantan, vorzugsweise 1-Bromadamantan, wird beispielsweise nach dem in Chem. Ber. 93, Seite 1629 (1959) beschriebenen Verfahren hergestellt.

Die Aufarbeitung des beim erfindungsgemäßen Verfahren anfallenden Reaktionsgemisches erfolgt zweckmäßigerweise durch Entfernung des Lösungsmittels, Extraktion des Rückstandes mit einem Solvens, in dem das Leitsalz unlöslich ist, vorzugsweise mit Ether oder Petrolether, und Verdampfung des Lösungsmittels, wodurch sich ein rohes diazinsubstituiertes 1-Adamantan der allgemeinen Formel I ergibt,

worin R die angegebenen Bedeutungen hat. Die Reinigung des jeweiligen Rohprodukts wird unter Anwendung üblicher Methoden durchgeführt, beispielsweise durch Kristallisation und Chromatographie.

Durch die erfindungsgemäße elektrochemische Umsetzung von 1-Bromadamantan mit den in der folgenden tabellarischen Aufstellung angegebenen weiteren Ausgangsmaterialien lassen sich beispielsweise die folgenden Endprodukte herstellen:

| Ausgangsmaterial | Endprodukt |
|---|---|
| Pyridazin oder 3,6-Dichlorpyridazin | 4-(Adamant-1-yl)-pyridazin |
| 4-Methylpyrimidin | 6-(Adamant-1-yl)-4-methylpyrimidin |
| 2-Aminopyrimidin | 4-(Adamant-1-yl)-2-aminopyrimidin |
| Pyrazin | 2-(Adamant-1-yl)-pyrazin |
| 2-Methylpyrazin | 6-(Adamant-1-yl)-2-methylpyrazin |
| 6-Amino-4-hydroxypyrimidin | 2-(Adamant-1-yl)-6-amino-4-hydroxypyrimidin (bisher nur nach einem anderen Verfahren hergestellt) |
| Pyrimidin | 2-(Adamant-1-yl)-pyrimidin<br>4-(Adamant-1-yl)-pyrimdin (bisher nach einem anderen Verfahren hergestellt) |

Durch Verwendung adamantylierter Diazine, wie von 4-(Adamant-1-yl)-pyrimidin, kann nach dem erfindungsgemäßen Verfahren ein zweiter Adamantylrest in den Heterocyclus eingeführt werden. Aus dem jeweiligen und nicht vorher adamantylierten Diazin können nach dem erfindungsgemäßen Verfahren jedoch auch diadamantylierte Diazine, wie 4,6-Di(adamant-1-yl)-pyrimidin, 2,6-Di(adamant-1-yl)-pyrazin oder 2,5-Di-(adamant-1-yl)-pyrazin, direkt durch Umsetzung mit einem 1-Halogenadamantan in nur einem Reaktionsschritt hergestellt werden.

Im Gegensatz zu den bekannten Verfahren für die Herstellung von diazinsubstituierten 1-Adamantanen, bei denen der Carbotricyclus nicht direkt mit diesen Substituenten funktionalisiert werden kann, ermöglicht das erfindungsgemäße Verfahren erstmals die direkte Einführung von Pyridazin-4-yl-, 2-Aminopyrimid-4(6)-yl-, 4-Methylpyrimid-4(6)-yl-oder Pyrazin-2(3,5,6)-yl-Resten und von bereits adamantylierten Diazinresten in das Adamantanmolekül, wodurch man neue diazinsubstituierte 1-Adamantane der angegebenen allgemeinen Formel (I) erhält, die virostatisch wirksam sind. Insbesondere hemmen das 2-(Adamant-1-yl)-pyrazin, 6-(Adamant-1-yl)-2-methylpyrazin, 6-Adamant-1-yl)-4-methylpyrimidin, 4-(Adamant-1-yl)-2-aminopyrimidin sowie das bekannte 4-(Adamant-1-yl)-pyrimidin Influenzaviren der verschiedenen Subtypen des Typs A, wobei einige dieser Verbindungen auch Influenzaviren vom Typ B hemmen, wobei die durchschnittliche Hemmung der Virusvermehrung 90 % bis 70 % beträgt (Virusertragstest mit dem Testsystem "Allantois-on-shell" (AOS)).

Die erfindungsgemäßen diazinsubstituierten 1-Adamantane unterscheiden sich von den bekannten und gegenüber Influenzaviren wirksamen 1-Amino-, 1-Aminosubstituierten- und 1-Aminomethylenadamantanen durch ihre andersartige heteroaromatische Substitution und durch eine überraschende Hemmwirkung gegenüber den humanpathogenen Influenzaviren vom Typ A und/oder vom Typ B. Im Gegensatz dazu ist bei den bekannten heteroaromatisch substituierten 1-Adamantanen lediglich eine antivi rale Wirksamkeit gegenüber dem nicht humanpathogenen Newcastle Disease Virus (NDV) bei zugleich hoher Zytotoxizität festgestellt worden.

Besonders hervorzuheben ist, daß die erfindungsgemäßen Verbindungen auch eine Hemmwirkung gegenüber Influenzaviren vom Typ B aufweisen. Verbindungen, die sich zur systemischen Therapie bei durch diesen Virustyp hervorgerufenen Infektionserkrankungen eignen, waren bisher gänzlich unbekannt. Auch das bekannte Rimantadin ist gegenüber Influenzaviren vom Typ B nicht spezifisch wirksam, wobei dessen antivirale Wirkung gegenüber Influenzaviren des Typs A von den erfindungsgemäßen Verbindungen bei einigen Subtypen sogar überschritten wird und in vielen Fällen in vergleichbarer Größenordnung liegt.

Die erfindungsgemäßen Verbindungen können in übliche pharmazeutische Formulierungen überführt werden, wie Tabletten, Kapseln, Dragees, Pillen, Granulate, Aerosole, Sirupe, Emulsionen, Suspensionen und Lösungen, wozu gegebenenfalls übliche inerte und nichttoxische pharmazeutisch geeignete Hilfsstoffe, Träger oder Lösungsmittel verwendet werden. Hierbei soll der Wirkstoff jeweils in einer Konzentration von etwa 0,5 bis 80 Gewichtsprozent der Gesamtmischung vorhanden sein, nämlich in Mengen, die zur Erzielung des jeweiligen Dosierungsspielraums ausreichen.

Beispiel 1

4,6-Di(adamant-1-yl)-pyrimidin

Man elektrolysiert 0,5 g Pyrimidin (0,006 mol) in einer Elektrolysezelle mit doppeltem G5-Frittendiaphragma in 200 ml wasserfreiem DMF/0,1 M Tetrabutylammoniumchlorid unter Zugabe von 1,7 g 1-Bromadamantan (0,008 mol) bei E = - 2,0 V (gegen Ag/0,1 M AgI) zwischen 300 mA zum Beginn und 20 mA zum Ende der Reaktion an einer 25 cm Mg-Kathode unter Durchleitung von trockenem $N_2$ als Schutzgas. Danach wird der Stromfluß unterbrochen und etwa 30 Minuten trockene Luft durch das Elektrolysat geleitet. Nach Zugabe von weiteren 1,7 g 1-Bromadamantan wird das Potential auf E = - 2,20 V eingestellt und solange weiter elektrolysiert, bis die Anfangsstromstärke von 250 mA auf 10 mA abgesunken ist (n = 10,6). Nach beendeter Reaktion wird das Hg abgetrennt, worauf die DMF-Lösung mit etwa dem gleichen Volumen Wasser versetzt und einige Zeit gekühlt wird. Das sich abscheidende kristalline Produktgemisch (0,93 g) wird abfiltriert, mit wenig eiskaltem Alkohol gewaschen und getrocknet. Filtrat und Waschflüssigkeit werden vereinigt, und das Lösungsmittelgemisch wird unter Vakuum verdampft. Der Rückstand wird mit Ether extrahiert, und die vereinigten Etherextrakte werden mit Wasser gewaschen und über $Na_2SO_4$ getrocknet, worauf das Lösungsmittel verdampft wird. Durch Auftrennung des Rohprodukts (1,21 g) an präparativen Kieselgelschichten mit Benzen : Essigsäureethylester (10 : 1) erhält man 0,57 g (Ausbeute = 27 % der Theorie, bezogen auf die eingesetzte Menge Pyrimidin) farbloses 4,6-Di-(adamant-1-yl)-pyrimidin mit einem Schmelzpunkt von 316 bis 318 °C.

| Elementaranalyse für $C_{24}H_{32}N_2$ (348,58): | | | |
|---|---|---|---|
| Berechnet: | C 82,69 | H 9,27 | N 8,04 |
| Gefunden : | C 82,53 | H 9,11 | N 8,20 |

Beispiel 2

6-(Adamant-1-yl)-4-methylpyrimidin

Man elektrolysiert 1 g (0,011 mol) 4-Methylpyrimidin analog zu Beispiel 1 in Gegenwart von 3 g (0,014 mol) 1-Bromadamantan bei E = -2,05 V (gegen Ag/0,1 M $AgBF_4$-Referenzelektrode) in trockenem Acetonitril/0,1 M Tetrabutylammoniumtetrafluorborat zwischen 300 mA zum Beginn und 35 mA zum Ende der Reaktion an einer Pb-Kathode (n = 5,6). Danach wird das Acetonitril unter Vakuum entfernt, der Rückstand mehrmals mit Ether extrahiert, getrocknet und die Etherschicht eingedampft. Das erhaltene Rohprodukt (1,55 g) wird durch präparative Schichtchromatographie an Kieselgel mit Benzen : Essigsäureethylester (1 : 1) gereinigt, wodurch man 1,25 g (Ausbeute = 50 % der Theorie) 6-(Adamant-1-yl)-4-methylpyrimidin in Form farbloser Kristalle mit einem Schmelzpunkt von 74 bis 75 °C erhält.

| Elementaranalyse für $C_{15}H_{20}N_2$ (228,37): | | | |
|---|---|---|---|
| Berechnet: | C 78,88 | H 8,85 | N 12,27 |
| Gefunden : | C 79,03 | H 8,70 | N 12,10 |

Beispiel 3

4-(Adamant-1-yl)-2-aminopyrimidin

Aus 1,6 g Rohprodukt, das nach der Elektrolyse von 1 g (0,011 mol) 2-Aminopyrimidin und 3 g (0,014 mol) 1-Bromadamantan bei einem Reduktionspotential von E = - 2,05 V (gegen Ag/0,1 M $AgBF_4$-Referenzelektrode) in wasserfreiem DMF/0,1 M Tetraethylammoniumtetrafluorborat und nach Aufarbeitung analog zu Beispiel 2 anfällt, wird druch chromatographische Auftrennung an präparativen Kieselgelschichten (Laufmittelsystem Benzen : Essigester (1 : 1)) 1,27 g (Ausbeute = 53 % der Theorie) erhalten. Das Rohprodukt wird dann aus wäßrigem Methanol umkristallisiert, wodurch man zu 4-(Adamant-1-yl)-2-aminopyrimidin in Form farbloser Kristalle gelangt, die bei 163 bis 164 °C schmelzen.

| Elementaranalyse für $C_{14}H_{19}N_3$ (229,36): | | | |
|---|---|---|---|
| Berechnet: | C 73,31 | H 8,37 | N 18,32 |
| Gefunden : | C 73,20 | H 8,31 | N 18,60 |

Beispiel 4

2-(Adamant-1-yl-)-pyrazin

In einer mit ungebranntem Tonmaterial zweifach geteilten Elektrolysezelle wird 1 g Pyrazin (0,012 mol) in absolutem Acetonitril/gesättigtem Tetraethylammoniumbromid unter Zugabe von 3 g 1-Bromadamantan analog zu Beispiel 1 potentiostatisch bei E = - 1,80 V (gegen Ag/0,1 M AgBr-Referenzelektrode) reduziert. Nachdem die Anfangsstromstärke von 300 mA auf 200 mA abgesunken ist (n = 2), wird die Elektrolyse abgebrochen. Hierauf wird das Hg abgetrennt, das Acetonitril unter Vakuum verdampft, der Rückstand mit Ether extrahiert, mit Wasser gewaschen, getrocknet und das Lösungsmittel entfernt. Das Rohprodukt (2,8 g) wird mittels präparativer Kieselgelschichtchromatographie im Laufmittelsystem Benzen : Essigsäureethylester (10 : 1) gereinigt, wodurch man 2,21 g (Ausbeute = 86 % der Theorie) farbloses kristallines 2-(Adamant-1-yl)-pyrazin erhält, das bei 61 bis 62 °C schmilzt.

| Elementaranalyse für $C_{14}H_{18}N_2$ (214,34): | | | |
|---|---|---|---|
| Berechnet: | C 78,45 | H 8,48 | N 13,07 |
| Gefunden : | C 78,29 | H 8,60 | N 12,89 |

Beispiel 5

2,6-Di(adamant-1-yl)-pyrazin und 2,5-Di(adamant-1-yl)-pyrazin

Man elektrolysiert 1 g Pyrazin (0,012 mol) und 6 g 1-Bromadamantan (0,028 mol) bei E = - 1,85 V, analog zu Beispiel 4, wobei die Elektrolyse nach einem Anfangsstromwert von 300 mA jedoch erst bei einem Endwert von 20 mA abgebrochen wird (n = 2,8). Durch entsprechende Aufarbeitung des Rohelektrolysats (2,44 g) analog zu Beispiel 3 und chromatographische Reinigung (präparative Kieselgelschicht, Benzen : Essigsäureethylester (10 : 1)) erhält man 0,43 g (Ausbeute = 21 % der Theorie) 2,6-Di(adamant-1-yl)-pyrazin und 0,25 g (Ausbeute = 12 % der Theorie) 2,5-Di(adamant-1-yl)-pyrazin jeweils in Form farbloser Kristalle, die bei 218 bis 220 °C und 259 bis 260 °C schmelzen.

| Elementaranalyse für $C_{24}H_{32}N_2$ (348,58) (2,6-Di(adamant-1-yl)-pyrazin): | | | |
|---|---|---|---|
| Berechnet: | C 82,69 | H 9,27 | N 8,04 |
| Gefunden : | C 82,45 | H 9,30 | N 8,20 |

| Elementaranalyse für $C_{24}H_{32}N_2$ (348,58) (2,5-Di(adamant-1-yl)-pyrazin): | | | |
|---|---|---|---|
| Berechnet: | C 82,69 | H 9,27 | N 8,04 |
| Gefunden : | C 82,50 | H 9,35 | N 8,22 |

Beispiel 6

6-(Adamant-1-yl)-2-methylpyrazin, 5-(Adamant-1-yl)-2-methylpyrazin und 3-(Adamant-1-yl)-2-methylpyrazin

Man elektrolysiert 1 g 2-Methylpyrazin (0,011 mol) analog zu Beispiel 1 in Gegenwart von 3 g 1-Bromadamantan bei einem konstanten Potential von E = - 1,85 V (gegen Ag/0,1 M AgI-Referenzelektrode) (n = 4,6). Das nach der Aufarbeitung anfallende harzige Produktgemisch (2,96 g) wird in wenig heißem Methanol/Chloroform aufgenommen und gekühlt. Die sich abscheidenden farblosen Kristalle des 6-(Adamant-1-yl)-2-methylpyrazins werden abfiltriert und aus Methanol umkristallisiert. Aus der methanolischen Mutterlauge wird durch Säulenchromatographie (Kieselgel; Benzen : Cyclohexan (3 : 1)) weiteres 6-(Adamant-1-yl)-2-methylpyrazin gewonnen. Aus dem Rückstand der verdampften Methanol/Chloroform-Lösung erhält man nochmals 6-(Adamant-1-yl)-2-methylpyrazin als farblosen Festsoff mit einem Schmelzpunkt von 55 bis 56 °C in einer Gesamtausbeute von 30 % der Theorie (0,75 g) durch Chromatographie an präparativen Kieselgelschichten (Benzen : Essigsäureethylester (6 : 1 )). Bei der Chromatographie fallen 5-Adamant-1-yl)-2-methylpyrazin (Ausbeute = 22 % der Theorie) und 3-(Adamant-1-yl)-2-methylpyrazin (Ausbeute = 11 % der Theorie) als Gemisch an, dessen Zusammensetzung durch NMR-Analyse bestimmt t werden kann.

| Elementaranalyse für $C_{15}H_{20}N_2$ (228,37): | | | |
|---|---|---|---|
| Berechnet: | C 78,88 | H 8,85 | N 12,27 |
| Gefunden : | C 78,60 | H 9,08 | N 12,28 |

Beispiel 7

4-(Adamant-1-yl)-pyridazin

Aus 1,41 g des Rohelektrolysats, das nach der elektrochemischen Reduktion von 1 g 3,6-Dichlorpyridazin (0,007 mol) unter Zusatz von 3 g 1-Bromadamantan bei einem Potential von E = - 1,85 V (gegen Ag/0,1 M AgI-Referenzelektrode) und Auf arbeitung entsprechend Beispiel 1 anfällt, erhält man nach Auftrennung des Produktgemisches mittels präparativer Kieselgelschichten mit Benzen : Essigsäureethylester : Methanol (15 : 3 : 2) 0,39 g (Ausbeute = 15 % der Theorie) 4-(Adamant-1-yl)-pyridazin in Form farbloser Kristalle mit einem Schmelzpunkt von 88 bis 90 °C.

| Elementaranalyse für $C_{14}H_{18}N_2$ (214,34) | | | |
|---|---|---|---|
| Berechnet: | C 78,45 | H 8,48 | N 13,07 |
| Gefunden : | C 78,70 | H 8,52 | N 12,89 |

Beispiel 8

4-(Adamant-1-yl)-pyrimidin

Man elektrolysiert 1 g (0,012 mol) Pyrimidin in einer Elektrolysezelle mit doppeltem G5-Frittendiaphragma in 200 ml wasserfreiem DMF/0,1 M Tetrabutylammoniumiodid unter Zugabe von 3,3 g (0,015 mol) 1-Bromadamantan an einer 25 $cm^2$ Hg-Kathode bei E = - 2,0 V (gegen Ag/0,1 M AgI-Referenzelektrode) zwischen 300 mA zum Beginn und 30 mA zum Ende der Reaktion (n = 3). Nach beendeter Reaktion wird das Hg abgetrennt und die DMF-Lösung mit etwa dem gleichen Volumen Wasser versetzt und einige Zeit gekühlt. Das sich abscheidende kristalline Produktgemisch (0,93 g) wird abfiltriert, mit wenig eiskaltem Ethanol gewaschen und getrocknet. Filtrat und Waschflüssigkeit werden vereinigt, und das Lösungsmittelgemisch unter Vakuum verdampft. Der Rückstand wird mit Ether extrahiert, und die vereinigten Etherextrakte werden mit Wasser gewaschen und über $Na_2SO_4$ getrocknet, worauf das Lösungsmittel verdampft wird. Das erhaltene Rohprodukt (1,27 g) wird mit dem eingangs isolierten Produktgemisch vereinigt (insgesamt 2,2 g) und über präparative Kieselgelschichten im Laufmittelsystem Benzen : Essigester (3 : 1) in die reinen Komponenten aufgetrennt. Hierdurch erhält man 1,8 g (Ausbeute = 70 % der Theorie) 4-(Adamant-1-yl)-pyrimidin in Form farbloser Kristalle, die bei 70,5 bis 71 °C schmelzen.

| Elementaranalyse für $C_{14}H_{18}N_2$ (214,34) (4-(Adamant-1-yl)-pyrimidin) | | | |
|---|---|---|---|
| Berechnet: | C 78,45 | H 8,48 | N 13,07 |
| Gefunden : | C 78,31 | H 8,63 | N 12,90 |

Beispiel 9

2-(Adamant-1-yl)-6-amino-4-hydroxypyrimidin

Man unterzieht 1 g (0,009 mol) 6-Amino-4-hydroxy-pyrimidin und 6 g 1-Bromadamantan (0,028 mol) in trockenem DMF/0,1 M Tetrabutylammoniumiodid-Lösung einer Elektroreduktion bei E = - 1,7 V (gegen Ag/0,1 M AgI). Durch analoge Aufarbeitung wie beim Beispiel 2 gelangt man zu 0,9 g Rohelektrolysat, das chromatographisch mittels einer Kieselgelsäule unter Verwendung von Benzen als Laufmittel gereinigt wird, wodurch man die Titelverbindung in Form einer farblosen viskosen Flüssigkeit (Ausbeute = 15 % der Theorie) erhält, die zur Kristallisation neigt und dünnschichtchromatographisch rein ist.

| Elementaranalyse für $C_{14}H_{19}N_3O$ (245,36) | | | | |
|---|---|---|---|---|
| Berechnet: | C 68,58 | H 7,82 | N 17,13 | O 6,52 |
| Gefunden : | C 68,81 | H 8,03 | N 17,01 | O 6,30 |

Tabelle 1

Virusertragstest; Testsystem "Allantois-on-shell (AOS)"

| Adamantyliertes Diazin | Antivirale Wirkung gegen Virusstamm | Wirkstoffkonzentration (Molarität) | Viruserstragshemmtest | | |
|---|---|---|---|---|---|
| | | | $ID_{50}$ *) ohne Wirkstoff (log 10) | $ID_{50}$ *) mit Wirkstoff (log 10) | Titerdifferenz (log 10) |
| 4-(Adamant-1-yl)-pyrimidin $C_{14}H_{18}N_2$ | Subtyp H2N2 A/Singapore 1/57 | $10^{-4}$ M | 6,5 | 5,17 | 1,33 |
| | | $10^{-4}$ M | 6,0 | 4,67 | 1,33 |
| | | $10^{-4}$ M | 6,83 | 5,33 | 1,5 |
| | | $10^{-4}$ M | 6,17 | 4,83 | 1,34 |
| | Subtyp H3N2 A/Philippines 2/82 | $10^{-4}$ M | 6,33 | 5,17 | 1,17 |
| | | $10^{-4}$ M | 5,83 | 5,17 | 0,66 |
| 6-(Adamant-1-yl)-4-methyl-pyrimidin $C_{15}H_{20}N_2$ | Typ A: Subtyp H1N1 A/Brasil 11/78 | $10^{-4}$ M | 4,83 | 3,83 | 1,0 |
| | | $10^{-4}$ M | 5,67 | 3,33 | 2,34 |
| | | $10^{-4}$ M | 5,67 | 3,0 | 2,67 |
| | Subtyp H2N2 A/Singapore 1/57 | $10^{-4}$ M | 6,66 | 5,17 | 1,49 |
| | | $10^{-4}$ M | 5,33 | 3,33 | 2,00 |
| | | $10^{-4}$ M | 4,33 | 3,33 | 1,0 |
| | A/Krasnodar 101/59 | $10^{-4}$ M | 5,0 | 2,5 | 2,5 |
| | Subtyp H3N2 A/Philippines 2/82 | $10^{-4}$ M | 6,08 | 5,0 | 1,08 |
| | | $10^{-4}$ M | 6,33 | 5,67 | 0,66 |
| | A/Hongkong 1/68 | $10^{-4}$ M | 4,75 | 3,83 | 0,92 |
| | Typ B B/Singapore 222/79 | $10^{-4}$ M | 6,33 | 5,67 | 0,66 |
| | | $10^{-4}$ M | 6,17 | 5,67 | 0,5 |

Tabelle 1 (Fortsetzung)

| Adamantyliertes Diazin | Antivirale Wirkung gegen Virusstamm | Wirkstoffkonzentration (Molarität) | Virusertragshemmtest $ID_{50}$ *) ohne Wirkstoff (log 10) | $ID_{50}$ *) mit Wirkstoff (log 10) | Titerdifferenz (log 10) |
|---|---|---|---|---|---|
| 4-(Adamant-1-yl)-2-amino-pyrimidin $C_{14}H_{19}N_3$ | Influenzaviren Typ A Subtyp H1N1 A/Brasil 11/78 | $0,5 \cdot 10^{-4}$ M | 5,30 | 2,50 | 2,8 |
| | | $0,5 \cdot 10^{-4}$ M | 5,33 | 3,33 | 2,0 |
| | H2N2 A/Singapore 1/57 | $0,5 \cdot 10^{-4}$ M | 5,55 | 4,0 | 1,55 |
| | | $0,5 \cdot 10^{-4}$ M | 5,50 | 3,83 | 1,67 |
| | Typ B B/Singapore 222/79 | $0,5 \cdot 10^{-4}$ M | 3,5 | 2,5 | 1,0 |
| | | $0,5 \cdot 10^{-4}$ M | 4,33 | 3,5 | 0,83 |
| 2-(Adamant-1-yl)-pyrazin $C_{14}H_{18}N_2$ | Subtyp H2N2 A/Singapore 1/57 | $10^{-4}$ M | 6,66 | 5,33 | 1,33 |
| | | $10^{-4}$ M | 6,0 | 4,83 | 1,17 |
| | | $10^{-4}$ M | 6,17 | 4,67 | 1,51 |
| | Subtyp H3N2 A/Philippines 2/82 | $10^{-4}$ M | 5,83 | 5,5 | 0,33 |
| | | $10^{-4}$ M | 6,33 | 5,5 | 0,83 |
| 6-(Adamant-1-yl)-2-methyl-pyrazin | Influenzaviren Typ A Subtyp H1N1 A/Brasil 11/78 | $0,5 \cdot 10^{-4}$ M | 5,30 | 3,33 | 1,97 |
| | | $0,5 \cdot 10^{-4}$ M | 5,33 | 3,67 | 1,66 |

Tabelle 1 (Fortsetzung)

| Adamantyliertes Diazin | Antivirale Wirkung gegen Virusstamm | Wirstoffkonzentration (Molarität) | Virusertragshemmtest $ID_{50}$ *) ohne Wirkstoff (log 10) | $ID_{50}$ *) mit Wirkstoff (log 10) | Titerdifferenz (log 10) |
|---|---|---|---|---|---|
| $C_{15}H_{20}N_2$ | H2N2 | $0,5 \cdot 10^{-4}$ M | 5,55 | 4,33 | 1,22 |
| | A/Singapore | $0,5 \cdot 10^{-4}$ M | 5,50 | 2,5 | 3,0 |
| | 1/57 | | | | |
| | H3N2 | $0,5 \cdot 10^{-4}$ M | 6,33 | 5,67 | 0,66 |
| | A/Hongkong | $10,5 \cdot 10^{-4}$ M | 5,83 | 5,5 | 0,33 |
| | 1/68 | $10^{-4}$ M | 5,67 | 5,33 | 0,34 |
| | | $10^{-4}$ M | 5,60 | 4,83 | 0,77 |
| | Typ B | $0,5 \cdot 10^{-4}$ M | 3,5 | 2,5 | 1,0 |
| | B/Singapore | $0,5 \cdot 10^{-4}$ M | 4,33 | 3,67 | 0,66 |
| | 222/79 | $10^{-4}$ M | 5,17 | 4,33 | 0,84 |
| | B/Hongkong | $10^{-4}$ M | 5,33 | 4,0 | 1,33 |
| | 5/72 | | | | |

*) $ID_{50}$: Infektionsdosis, bei der 50 % des Substrats infiziert werden: Infektionstiter

Tabelle 2

| Antivirale Wirkung von Rimantadin (α-Methyl-1-adamantanmethylaminhydrochlorid) gegen Influenzaviren Typ A und Typ B | | | | |
|---|---|---|---|---|
| Testsystem: Allantois-on-shell(AOS) | | | | |
| Antivirale Wirkung gegen Virusstamm | Wirkstoffkonzentration in µg/ml (Molarität) | Virusertragshemmtest | | |
| | | $ID_{50}$ (log 10) ohne Wirkstoff | $ID_{50}$ (log 10) mit Wirkstoff | Titerreduktion (log 10) |
| Influenzavirus | 10 µg/ml | 5,5 | 4,0 | 1,5 |
| Typ A | (etwa 0,5 x $10^{-4}$) | 4,83 | 3,33 | 1,5 |
| Subtyp | 20 µg/ml | 4,67 | 3,17 | 1,5 |
| H1N1 | (etwa $10^{-4}$) | 5,17 | 3,33 | 1,84 |
| A/Taiwan | | | | |
| 1/86 | | | | |
| Subtyp | 10µg/ml | 5,0 | 2,5 | 2,5 |
| H2N2 | (etwa 0,5 x $10^{-4}$) | 5,0 | 2,5 | 2,5 |
| A/Singapore | 20 µg/ml | 5,67 | 2,5 | 3,17 |
| 1/57 | (etwa $10^{-4}$) | 5,83 | 2,5 | 3,33 |
| Subtyp | 10 µg/ml | 6,83 | 5,28 | 1,55 |
| H3N2 | (etwa 0,5 x $10^{-4}$) | 6,83 | 5,36 | 1,47 |
| A/Hongkong | 20 µg/ml | 6,17 | 2,83 | 3,34 |
| 1/68 | (etwa $10^{-4}$) | 5,67 | 3,0 | 2,67 |
| A/Philippines | 20 µg/ml | 5,17 | 5,0 | 0,17 |
| 2/82 | (etwa $10^{-4}$) | 5,0 | 4,83 | 0,17 |
| Influenzavirus | 10 µg/ml | 6,83 | 7,17 | -0.34 |
| Typ B | (etwa 0,5 x $10^{-4}$) | 6,83 | 6,83 | 0 |
| B/Ann | 20 µg/ml | 6,17 | 6,17 | 0 |
| Arbor 1/86 | (etwa $10^{-4}$) | 6,33 | 6,33 | 0 |

**Ansprüche**

1. Diazinsubstituierte 1-Adamantane der allgemeinen Formel I

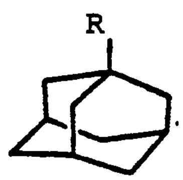 (I)

,

worin R entweder für einen unsubstituierten oder einen einfach in möglichen Positionen durch Alkyl, Alkoxy, Aryl, Amino, Acylamino, Hydroxy, Adamant-1-yl, Halogen oder Pseudohalogen substituierten oder für einen mehrfach in möglichen Positionen gleich oder verschieden durch Alkyl, Alkoxy, Aryl, Amino, Acylamino, Hydroxy, Halogen oder Pseudohalogen substituierten oder einen mehrfach in möglichen Positionen gleich oder verschieden durch Alkyl, Alkoxy, Aryl, Amino, Acylamino, Hydroxy, Halogen oder Pseudohalogen und durch Adamant-1-yl substituierten Pyridazinrest oder Pyrazinrest steht,

oder worin R einen unsubstituierten oder einen einfach in möglichen Positionen durch Alkoxy, Aryl, Acylamino, Halogen oder Pseudohalogen oder durch 2-Amino, 2- oder 4(6)-Alkyl oder 2- oder 4(6)-(Adamant-1-yl) substituierten oder für einen mehrfach in möglichen Positionen gleich oder verschieden

durch Alkyl, Alkoxy, Aryl, Acylamino, Halogen oder Pseudohalogen substituierten oder einen mehrfach in möglichen Positionen gleich oder verschieden durch Alkyl, Alkoxy, Aryl, Acylamino, Halogen oder Pseudohalogen und durch Adamant-1-yl substituierten Pyrimidinrest bedeutet.

2. Diazinsubstituierte 1-Adamantane der allgemeinen Formel I nach Anspruch 1, dadurch gekennzeichnet, daß die möglichen Positionen der Substitution im Pyridazinrest die Kohlenstoffatome 3 und/oder 4 und/oder 5 und/oder 6, im Pyrazinrest die Kohlenstoffatome 2 und/oder 3 und/oder 5 und/oder 6 und im Pyrimidinrest die Kohlenstoffatome 2 und/oder 4 und/oder 5 und/oder 6 sind, daß Alkyl jeweils für unverzweigtes $C_nH_{2n+1}$ mit n gleich 1 bis 4 steht, daß Alkoxy jeweils unverzweigtes $C_nH_{2n+1}O$- mit n gleich 1 bis 4 bedeutet, daß Aryl jeweils Phenyl oder durch einen oder mehrere Elektronendonatoren substituiertes Phenyl ist, daß Amino für $H_2N$-, $CH_3NH$-, $C_2H_5NH$-, $(CH_3)_2N$- oder $(C_2H_5)_2N$-, daß Acylamino für $CH_3CO$ NH-, $C_2H_5CONH$-, $C_6H_5CONH$- oder $C_6H_5CH_2CONH$- steht, daß Halogen Fluor, Chlor, Brom oder lod ist, und daß Pseudohalogen Cyano bedeutet.

3. Diazinsubstituierte 1-Adamantane der allgemeinen Formel I nach Anspruch 1 und 2, dadurch gekennzeichnet, daß diese Verbindungen 1-Adamantane sind, die durch Pyridazin-4(5)-yl, Pyrazin-2(3,5,6)-yl, 2-Methylpyrazin-6(5,3)-yl, 6-(Adamant-1-yl)-pyrazin-2-yl, 5-(Adamant-1-yl)-pyrazin-2-yl, 4(6)-Methylpyrimidin-6(4)-yl, 4(6)-(Adamant-1-yl)-pyrimidin-6(4)-yl oder 2-Aminopyrimidin-6(4)-yl substituiert sind.

4. Arzneimittel, insbesondere Virostatikum, vor allem Mittel gegen Influenzaviren, dadurch gekennzeichnet, daß es mindestens eine Verbindung der allgemeinen Formel I nach Anspruch 1 als Wirkstoff gegebenenfalls zusammen mit üblichen Hilfsstoffen und/oder Trägern enthält.

5. Arzneimittel nach Anspruch 4, dadurch gekennzeichnet, daß es eine Verbindung der allgemeinen Formel I, worin R Pyrimidin-4(6)-yl, 4,6-Dihydroxypyrimidin-2-yl, 6-Amino-4-hydroxypyrimidin-2-yl oder 5-Methylpyrimidin-4(6)-yl bedeutet, als Wirkstoff enthält.

6. Verfahren zur Herstellung der diazinsubstituierten 1-Adamantane der allgemeinen Formel I nach Anspruch 1, dadurch gekennzeichnet, daß ein 1-Halogenadamantan, vorzugsweise 1-Bromadamantan,mit einem unsubstituierten oder einfach in möglichen Positionen durch Alkyl- Alkoxy, Aryl, Amino, Acylamino, Hydroxy, Adamant-1-yl, Halogen oder Pseudohalogen substituierten oder einem mehrfach in möglichen Positionen gleich oder verschieden durch Alkyl, Alkoxy, Aryl, Amino, Acylamino, Hydroxy, Halogen oder Pseudohalogen substituierten oder einen mehrfach in möglichen Positionen gleich oder verschieden durch Alkyl, Alkoxy, Aryl, Amino, Acylamino, Hydroxy, Halogen oder Pseudohalogen und durch Adamant-1-yl substituiertes Pyridazin mit freier oder mit durch Chlor, Brom oder lod substituierter Stellung 3(6) und/oder Stellung 4(5) oder Pyrazin mit freier oder mit durch Chlor, Brom oder lod substituierter Stellung 2 oder Pyrimidin mit freier oder mit durch Chlor, Brom oder lod substituierter Stellung 2 und/oder Stellung 4(6) vermischt und in einem wasserfreien Lösungsmittel in Gegenwart eines Leitsalzes unter Schutzgasatmosphäre elektrochemisch umgesetzt wird.

7. Verfahren nach Anspruch 6, dadurch gekennzeichnet, daß als Reaktant zur Einführung des Restes R Pyridazin, 3,6-Dichlorpyridazin, Pyrazin, 2-Methylpyrazin, 2-(Adamant-1-yl)-pyrazin, 4(6)-Methylpyrimidin, 2-Aminopyrimidin, 4(6)-(Adamant-1-yl)-pyrimidin oder 6-Amino-4-hydroxypyrimidin verwendet wird.

8. Verfahren nach einem der Ansprüche 6 oder 7, dadurch gekennzeichnet, daß die Umsetzung in Dimethylformamid, Acetonitril, Propylencarbonat oder Methanol als wasserfreiem Lösungsmittel durchgeführt wird.

9. Verfahren nach einem der Ansprüche 6 bis 8, dadurch gekennzeichnet, daß die Umsetzung in Gegenwart eines Tetraalkylammoniumsalzes, vorzugsweise von Tetraethylammoniumiodid, Tetraethylammoniumbromid, Tetraethylammoniumperchlorat, Tetraethylammoniumtetrafluorborat, Tetrabutylammoniumiodid, Tetrabutylammoniumbromid, Tetrabutylammoniumperchlorat oder Tetrabutylammoniumtetrafluorborat, als Leitsalz durchgeführt wird.

Europäisches Patentamt

# EUROPÄISCHER RECHERCHENBERICHT

Nummer der Anmeldung

EP 89 11 6317

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A,D | JOURNAL OF CHEMICAL EDUCATION Band 50, Nr. 11, 1973, Seiten 780,781; J. S. WISHNOK: "Medicinal Properties of Adamantane Derivatives" * Seite 781, Formel VIII * --- | 1-3 | C 07 D 239/26<br>C 07 D 239/42<br>C 07 D 241/12<br>C 07 D 237/08<br>C 07 D 239/46<br>A 61 K 31/505<br>A 61 K 31/495<br>A 61 K 31/50<br>C 25 B 3/10 |
| A,D | JOURNAL OF MEDICINAL CHEMISTRY Band 13, Nr. 6, 1970, Seiten 1170-1172; J. P. JONAK et al.: "Synthesis and Biological Activity of Some 5-(1-Adamantyl)pyrimindines" * Seite 1171, Tabelle I, Schema I * --- | 1-3 | |
| A,D | JOURNAL OF MEDICINAL CHEMISTRY Band 14, Nr. 5, 1971, Seiten 408-411; J.P. JONAK et al.: "Synthesis and Biological Activity of Some 5-(1-Adamantyl)pyrimidines 2" * Seite 408, Zusammenfassung; Seite 409, Tabelle 1 * --- | 1-3 | |
| A | CHEMICAL ABSTRACTS Band 77, Nr. 13, 25. September 1972, Seite 467, Zusammenfassung Nr. 88532y, Columbus, Ohio, USA; & CS-142695 --- | 1,4 | **RECHERCHIERTE SACHGEBIETE (Int. Cl.5)**<br>C 07 D 237/00<br>C 07 D 239/00<br>C 07 D 241/00 |
| A | CHEMICAL ABSTRACTS Band 77, Nr. 17, 23. Oktober 1972, Seite 455, Zusammenfassung nr. 114428r, Columbus, Ohio, USA; & CS-142696 --- | 1,4 | |
| A,D | JOURNAL OF MEDICINAL CHEMISTRY Band 14, Nr. 6, 1971, Seiten 535-543; P.E. ALDRICH et al.: "Antiviral Agents. 2. Structure-Activity Relationships of Compounds Related to 1-Adamantanamine" --- -/- | 1,4 | |

Der vorliegende Recherchenbericht wurde für alle Patentansprüche erstellt

| Recherchenort | Abschlußdatum der Recherche | Prüfer |
|---|---|---|
| BERLIN | 23-11-1989 | HASS C V F |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)

Europäisches Patentamt

# EUROPÄISCHER RECHERCHENBERICHT

Nummer der Anmeldung

EP 89 11 6317

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | US-A-4 252 954 (R. F. ABDULLA et al.) * Ansprüche 1,3,4,8 * | 1,4 | |
| A | US-A-4 670 437 (R. F. ABDULLA) * Spalte 6, Tabelle, viertletzte und drittletzte Verbindung; Spalte 6, Zeile 49 - Spalte 7, Zeile 36 * | 1,4 | |
| A | CHEMICAL ABSTRACTS Band 70, Nr. 5, 3. Februar 1969, Seite 1995, Zusammenfassung Nr. 20025k, Columbus, Ohio, USA; C. RUNTI et al.: "Possible antiviral compounds. V. Higher N-acyl derivatives of s-triazine bases" & Int. Congr. Chemother., Proc., 5th 1967, Nr. 6, Seiten 551-556 | 1,4 | |
| A | CHEMICAL ABSTRACTS Band 82, Nr. 17, 28. April 1975, Seite 28, Zusammenfassung 106315q, Columbus, Ohio, USA; V.I. IL'ENKO et al.: "Chemoprophylactic activity of amantadine derivatives" & Khimioprofil. Khimioter. Grippa, Mater. Vses. Simp., 1st 1971 (Pub. 1972), Seiten 40-44 | 1,4 | RECHERCHIERTE SACHGEBIETE (Int. Cl.5) |
| A | CHEMICAL ABSTRACTS Band 84, Nr. 3, 19. Januar 1976, Seite 86, Zusammenfassung Nr. 12961m, Columbus, Ohio, USA; F. SZTARICSKAI et al.: "Synthesis and in vitro virucidal properties of recent 1-substituted adamantane derivatives" & Pharmazie 1975, Band 30, Nr. 9, Seiten 571-581 -/- | 1,4 | |

Der vorliegende Recherchenbericht wurde für alle Patentansprüche erstellt

| Recherchenort | Abschlußdatum der Recherche | Prüfer |
|---|---|---|
| BERLIN | 23-11-1989 | HASS C V F |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

Europäisches Patentamt

# EUROPÄISCHER RECHERCHENBERICHT

Nummer der Anmeldung

EP 89 11 6317

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | CHEMICAL ABSTRACTS Band 99, Nr. 18, 31. Oktober 1983, Seite 531, Zusammenfassung Nr. 148365n, Columbus, Ohio, USA; U. HESS et al.: "Electrochemical adamantylation of stilbenes" & Pharmazie 1983, Band 38, Nr. 4, Seiten 669-678 --- | 6,8 | |
| A | CHEMICAL ABSTRACTS Band 99, Nr. 8, 22. August 1983, Seite 445, Zusammenfassung Nr. 60833c, Columbus, Ohio, USA; U. HESS et al.: "Electrochemical adamantylation of styrylpyridines" & J. Prakt. Chem. 1983, Band 325, Nr. 2, Seiten 301-308 ----- | 6,8 | |
| | | | RECHERCHIERTE SACHGEBIETE (Int. Cl.5) |

Der vorliegende Recherchenbericht wurde für alle Patentansprüche erstellt

| Recherchenort | Abschlußdatum der Recherche | Prüfer |
|---|---|---|
| BERLIN | 23-11-1989 | HASS C V F |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)